# EUROPEAN PATENT APPLICATION

(11) **EP 1 992 300 A2**
(43) Date of publication of application: **19.11.2008**
(21) Application number: 08251690.7
(22) Date of filing: 14.05.2008
(51) Int. Cl.: A61B 18/14

(54) **A catheter sheath**

(30) Priority: 18.05.2007 US 930923 P; 17.07.2007 US 961017 P
(71) Applicant: CathRx Ltd, Eveleigh, NSW 1430 (AU)
(72) Inventor: Ogle, David, Cowan, New South wales 2081 (AU); Anderson, Neil Lawrence, Roseville, New South Wales 2069 (AU); Milijasevic, Zoran, Bayview, New South Wales 2104 (AU)
(74) Representative: Burt, Matthew Thomas

(57) **Abstract**

A catheter sheath 12 includes a tubular member 14 defining a lumen 18, a distal region of the tubular member 14 defining a plurality of discrete elements 28. The discrete elements 28 are displaceable between a first position in which the discrete elements 28 extend parallel to a longitudinal axis of the tubular member 14 and a second position in which the discrete elements 28 project outwardly transverse to the longitudinal axis of the tubular member 14. A plurality of electrical conductors 30 is associated with the tubular member 14, the conductors 30 projecting into the distal region of the tubular member 14. A sleeve is about the tubular member 14, the sleeve and the tubular member 14 being displaceable axially with respect to each other for effecting displacement of the discrete elements 28 between their first and second positions. At least one electrode 36 is carried by at least one of the discrete elements 28, the at least one electrode 36 being in an operative position when the discrete elements 28 are in their second position.

## Description

### Field

This invention relates generally to the field of catheters and, more particularly, to a catheter sheath for a catheter assembly and to a catheter assembly including the catheter sheath.

### Background

In the field of heat treatment of tissue, it is desirable if the device heating the tissue is in contact only with the tissue being treated and not surrounding tissue or bodily fluids. This minimises the power required to heat the tissue and also minimises unnecessary damage to other tissue, structures or fluid.

In addition, it is often necessary to overcome tissue irregularities at a site in a patient's body being heat treated. An example where a site in a patient's body is subjected to heat treatment is in the treatment of heart arrhythmias where tissue is ablated in an effort to cure the arrhythmia. The tissue is ablated to create a lesion to block the electrical impulses causing the arrhythmia. To ensure that a lesion of adequate depth is formed, it is desirable that the ablating electrode makes good contact with the tissue.

Other examples of the use of heat treatment at a site in a patient's body include treatment of Parkinson's disease, tumour ablation, endometriosis and pain management.

Still further, in the treatment of arrhythmia, it may be necessary to ablate over a reasonably wide area in an attempt to cure the arrhythmia. It would be beneficial to be able to obtain such larger ablated areas with minimum manipulation of the catheter when in position at the site to be treated.

There is therefore a need for a catheter sheath and a catheter assembly which meets these needs. Such a catheter sheath and catheter assembly could also be useful in other applications, for example, pacing, sensing or defibrillation.

### Summary

According to a first aspect of the invention, there is provided a catheter sheath which includes
a tubular member defining a lumen, a distal region of the tubular member defining a plurality of discrete elements, the discrete elements being displaceable between a first position in which the discrete elements extend parallel to a longitudinal axis of the tubular member and a second position in which the discrete elements project outwardly transverse to the longitudinal axis of the tubular member;
a plurality of electrical conductors associated with the tubular member, the conductors projecting into the distal region of the tubular member to be made fast with at least one of the discrete elements;
a sleeve mounted about the tubular member, the sleeve and the tubular member being displaceable axially with respect to each other for effecting displacement of the discrete elements between their first and second positions; and
at least one electrode carried by the at least one of the discrete elements, the at least one electrode being in an operative position when the discrete elements are in their second position.

The tubular member may be of a settable material so that the discrete elements can be set in one of their first position and their second position as a rest position. The tubular member may be of a heat settable material such as a shape memory alloy. For example, the tubular member may be of Nitinol. Preferably, the tubular member is mounted on a tube of a flexible material such as a suitable bio-compatible synthetic plastics material. For example, the tube may be of a polyetheretherketone (PEEK) material.

In one embodiment, the rest position of the discrete elements may be the second position and the sleeve may be axially displaceable relative to the tubular member, displacement of the sleeve towards a distal end of the tubular member displacing the discrete elements to their first position and withdrawal of the sleeve in a proximal direction exposing the discrete elements and allowing the discrete elements to adopt their second position.

In another embodiment, the rest position of the discrete elements may be the first position, the sleeve having complementary discrete components which lie in register with the discrete elements of the tubular member, each discrete element being secured to its associated distal component so that proximal displacement of the sleeve relative to the tubular member causes the discrete elements to be displaced to their second position.

Each discrete element may be in the form of a strut, each strut supporting at least one electrode. The distal region of the tubular member may therefore be crenelated having deep crenelations to define the struts. In an embodiment, the distal region of the sleeve may be similarly crenelated to define the discrete components. By "deep" is meant that each strut has a length greater than twice the width of the strut.

The at least one electrode of each strut may be carried on a carrier which is mounted on the strut. Each carrier may be a sheath-like structure which fits over its associated strut.

In an embodiment, the at least one electrode may be arranged on an operatively inner surface of its associated carrier so that, when the discrete elements are in their first position, the electrodes are contained within the interior of the tubular member. In another embodiment, the at least one electrode may be arranged on an operatively outer surface of is associated carrier so that, when the discrete elements are in their first position, the electrodes are contained on an outer surface of the tubular member.

The electrical conductors may be arranged in groups of conductors, a group of conductors being associated with each discrete element. More particularly, each group of conductors may comprise four conductors, a pair of conductors being used for the delivery of energy, such as RF energy for ablation, to its associated electrode and a pair of conductors for temperature sensing associated with that electrode. Each group of conductors may be configured as ribbon cable extending through the lumen of the tubular member.

At least one of the tubular member and the sleeve may define a deflection zone arranged proximally of the discrete elements of the tubular member. The catheter sheath may include an actuator which acts on the deflection zone for controlling deflection of the distal region of the tubular member.

According to a second aspect of the invention, there is provided a catheter assembly which includes
a catheter sheath as described above; and
a support member arranged within the lumen of the tubular member.

The catheter assembly may include an end electrode carried on a distal end of the support member and arranged in alignment with the distal end of the tubular member.

The support member may be displaceably arranged in the lumen of the tubular member, the arrangement being such that, when the discrete elements of the tubular member are in their first position, the end electrode is arranged distally of the discrete elements and, when the discrete elements are in their second position, the support member is displaced proximally so that the end electrode lies substantially in a plane in which the discrete elements lie.

A flexible cylindrical member may be interposed between the end electrode and the distal end of the support member to create increased flexibility at the distal region

A seal may be arranged about the support member in the region of the discrete elements of the tubular member so that, when the discrete elements of the tubular member are in their first position, they seat about the seal to inhibit the ingress of foreign matter into the lumen of the tubular member.

### Brief Description of Drawings

Fig. 1 shows a three dimensional view of an embodiment of a catheter assembly in a closed configuration;
Fig. 2 shows a three dimensional view of the catheter assembly in an open configuration;
Fig. 3 shows an end view of the catheter assembly in its open configuration;
Fig. 4 shows a three dimensional, exploded view of a part of the catheter assembly in its open configuration;
Fig 5 shows a schematic, cross sectional view, from a first direction, of a part of the catheter assembly with some parts omitted for the sake of clarity;
Fig. 6 shows a schematic, cross sectional view, from the opposite direction, of the catheter assembly;
Fig. 7 shows a three dimensional view of a tubular member of the catheter assembly;
Fig. 8 shows a side view of the tubular member;
Fig. 9 shows an end view of the tubular member;
Fig. 10 shows an end view, on an enlarged scale, of the part of the tubular member surrounded by circle 'A' in Fig. 9;
Fig. 11 shows a three dimensional view of another embodiment of a catheter assembly;
Fig. 12 shows a three dimensional view of a further embodiment of a catheter assembly;
Fig. 13 shows a three dimensional view, on an enlarged scale, of the part of the catheter assembly surrounded by circle 'B' in Fig. 12; and
Fig. 14 shows a three dimensional view of yet a further embodiment of a catheter assembly.

### Detailed Description of Exemplary Embodiments

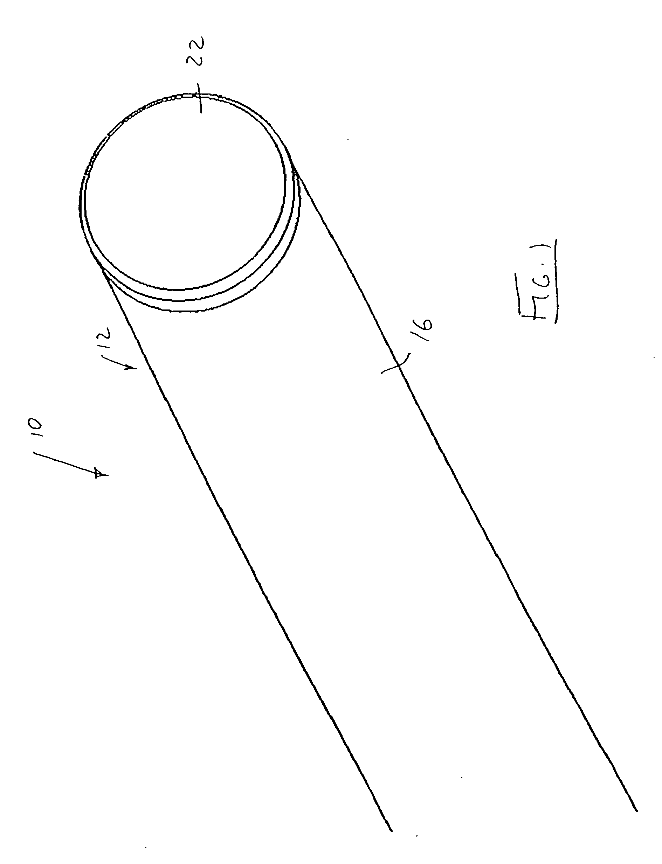

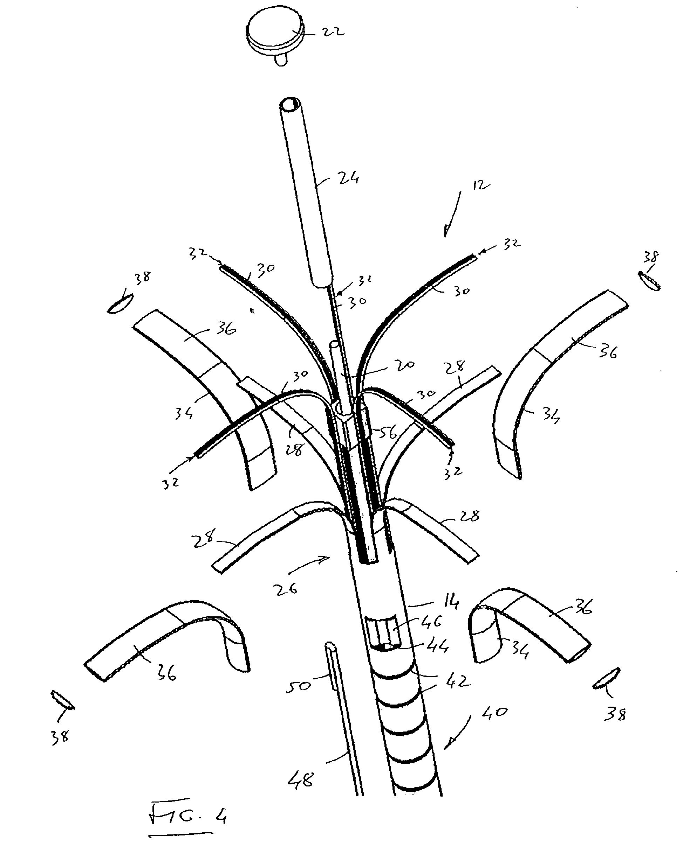

In the drawings, reference 10 generally designates an embodiment of a catheter assembly. The catheter assembly 10 comprises a catheter sheath 12. The catheter sheath 12 has a tubular member 14 (Fig. 4) surrounded by a sleeve 16 (Fig. 1). The sleeve 16 and tubular member 14 are displaceable relative to each other.

The tubular member 14 defines a lumen 18 in which a support member 20 (Fig. 4) is displaceably received. The support member 20 supports an end electrode 22 via a flexible cylindrical member 24, as will be described in greater detail below.

A distal region 26 of the tubular member 14 defines a plurality of discrete elements in the form of struts 28. In this regard, it is to be noted that the tubular member 14 is of a settable material. More particularly, the tubular member 14 is of a shape memory alloy such as, for example, Nitinol. In this embodiment, the tubular member 14 is pre-set so that, as illustrated in Fig. 4 of the drawings, in their rest position, the struts 28 project outwardly transversely to a longitudinal axis of the tubular member 14. More particularly, the struts 28 extend substantially radially outwardly to lie in a plane which is perpendicular to a longitudinal axis of the tubular member 14.

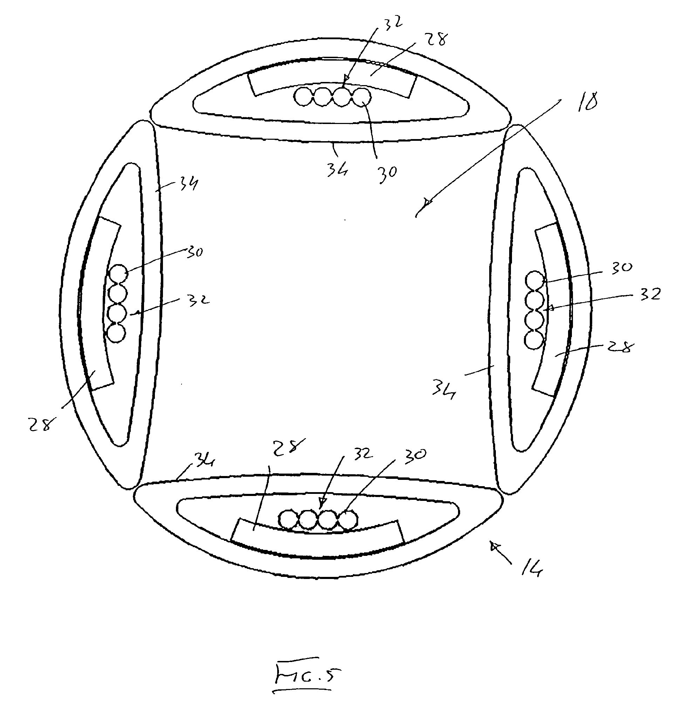

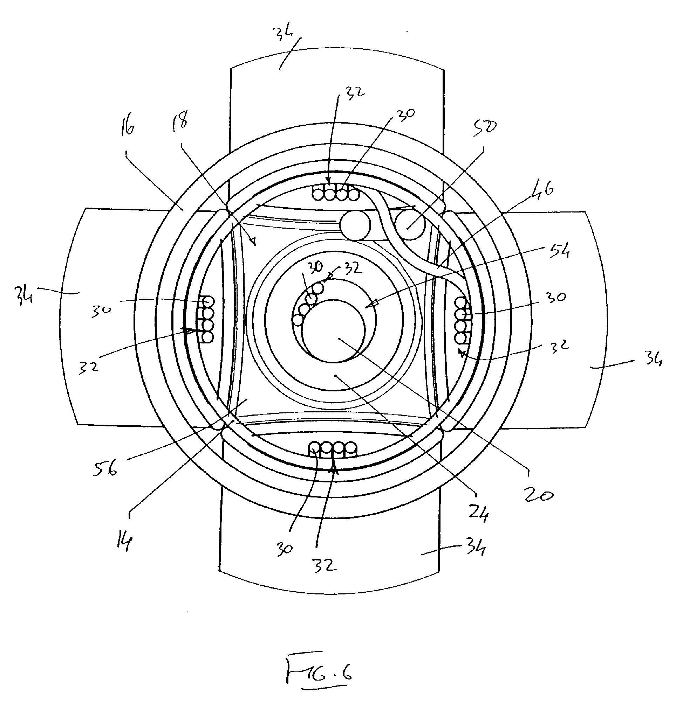

A plurality of conductors 30 is associated with each strut 28 as well as with the end electrode 22, as will be described in greater detail below. The conductors 30 are arranged in groups 32 implemented in the form of ribbon cable. As illustrated more clearly in Figs. 5 and 6 of the drawings, the groups 32 of conductors 30 extend through the lumen 18 of the tubular member 14.

Each strut 28 has a sheath-like carrier 34 associated with it. An electrode 36 is carried on an operatively inner surface of each carrier 34. In use, each group 32 of conductors 30 is electrically connected to, and mechanically fast with, its associated electrode 36. Each group 32 of conductors 30 comprises four conductors 30. Two of the conductors 30 are used for the delivery of energy, such as radio frequency (RF) energy, to the associated electrode 36 for ablation purposes. In addition, two conductors 30 are provided in the form of a Constantin/copper wire pair for a thermocouple for temperature sensing associated with the electrodes 22 and 36.

Each carrier 34 is a sliding fit over its associated strut 28. A distal end of the carrier 34 is closed off by an end cap 38 to inhibit the ingress of detritus or bodily fluids into the lumen 18 of the tubular member 14.

The catheter sheath 12 includes a deflection zone 40 which, in this embodiment, is defined by the tubular member 14. The deflection zone 40 comprises a plurality of longitudinally spaced slots 42 formed in the tubular member 14, for example, by laser cutting. The slots 42 extend approximately two thirds to three quarters of the way about a periphery of the tubular member 14 to leave a longitudinally extending spine zone (not shown in this embodiment) about which the tubular member 14 can be deflected.

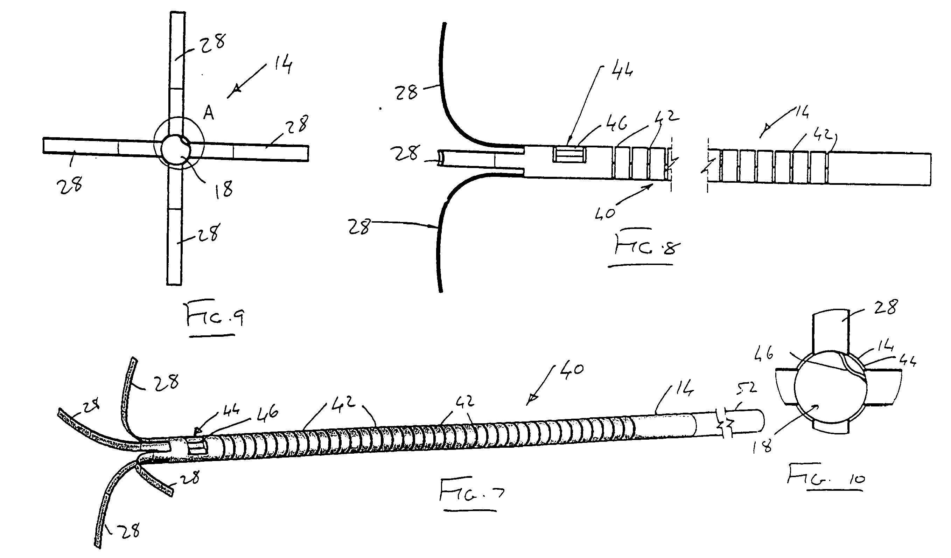

The tubular member 14 includes a mounting window 44 defined distally of the slots 42. The mounting window 44 has a mounting formation 46 arranged in it. As illustrated more clearly in Fig. 10 of the drawings, the mounting formation 46 is an indented portion of a wall of the tubular member 14 and lies within the lumen 18 of the tubular member 14.

An actuator, in the form of a pull wire, 48 is received within the lumen 18 of the tubular member 14. A distal end of the pull wire 48 has a hairpin hook 50 which engages the mounting formation 46 as shown more clearly in Fig. 6 of the drawings. Urging the pull wire 48 in a proximal direction causes bending of the distal region of the tubular member 14 in the deflection zone 40 about the spine.

As described above, the end electrode 22 is mounted on the support member 20 via the flexible cylindrical member 24. The flexible cylindrical member 24 is of a sufficient length that it extends through the deflection zone 40 of the tubular member 14 and terminates proximally of the deflection zone 40. Thus, when it is desired to deflect the tubular member 14 of the catheter sheath 12, the flexible cylindrical member 24 facilitates deflection of the tubular member 14 in the deflection zone 40. The flexible cylindrical member 24 is of polyetheretherketone (PEEK) material or a polyether block amide material such as Pebax®. The conductors 30 of the group 32 of conductors associated with the end electrode 22 protrude through a bore 54 (Fig. 6) of the flexible cylindrical member 24.

The tubular member 14, is, in use, mounted on a tube 52, indicated schematically in Fig. 7 of the drawings. The tube 52 is also of a flexible, synthetic plastics material such as a PEEK or Pebax®.

A seal 56 is mounted about the flexible cylindrical member 24. The seal 56 seats sealingly about an outer periphery of the flexible cylindrical member 24 and also abuts against the carriers 34, when the carriers 34 are in the position shown in Fig. 1 of the drawings, i.e. constrained by the sleeve 16, to inhibit the ingress of detritus or bodily fluid into the lumen 18 of the tubular member 14.

In use, to form the tubular member 14, crenelations are formed in the distal region of the tubular member 14 to define the struts 28. The struts 28 are heat set in their splayed configuration as shown in Fig. 4 of the drawings. This configuration is a second, operative configuration of the struts 28. It is to be noted that, in this embodiment, the electrodes 36 are carried on an operatively inner surface of each they extend parallel to the longitudinal axis of the tubular member 14 (as shown in Fig. 1 of the drawings), the electrodes 36 are contained within the interior of the tubular member 14. The struts 28 are retained in this position by sliding the sleeve 16 towards the distal end of the catheter assembly 10 until a distal end of the sleeve 16 abuts the end electrode 22.

The catheter assembly 10 is inserted, in this configuration, into the vasculature of a patient to enable the catheter assembly 10 to be steered to a desired site in the patient's body. More particularly, the catheter assembly 10 is steered to a desired site in a patient's heart to enable ablation therapy to be carried out on the heart.

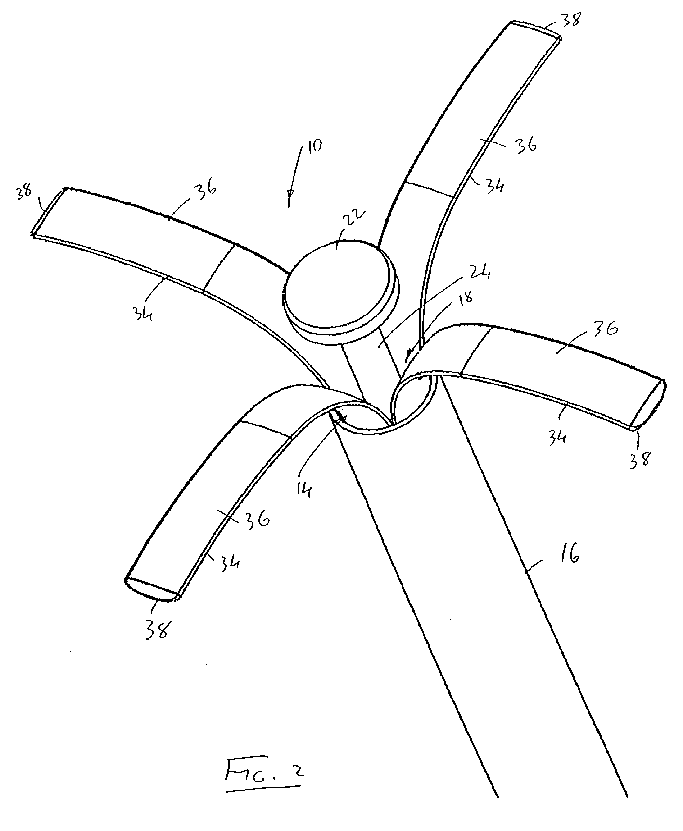

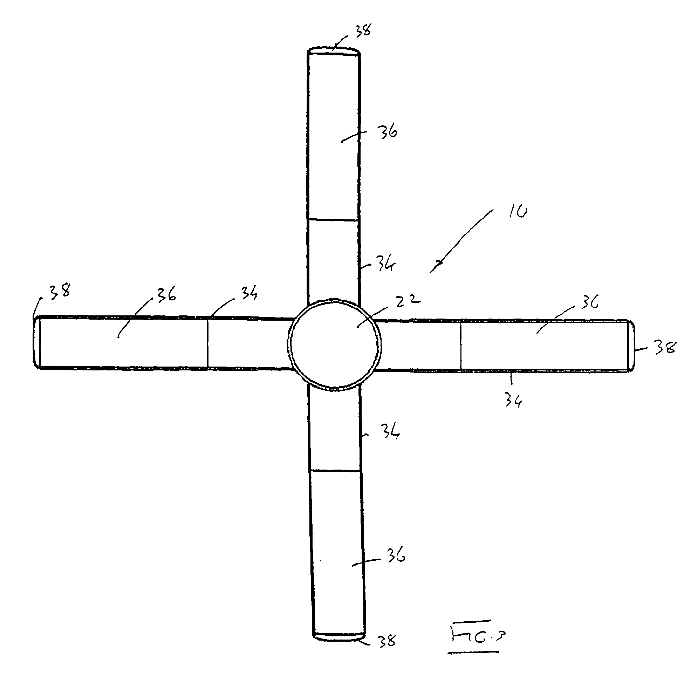

At the desired site in the patient's body, the sleeve 16 is withdrawn proximally relative to the tubular member 14 to expose the struts 28. The struts 28 then adopt their splayed, second position shown in Figs. 2 and 3 of the drawings and the electrodes 36 are exposed. The electrodes 36 can be urged against tissue in the patient's heart for enabling ablation therapy to take place.

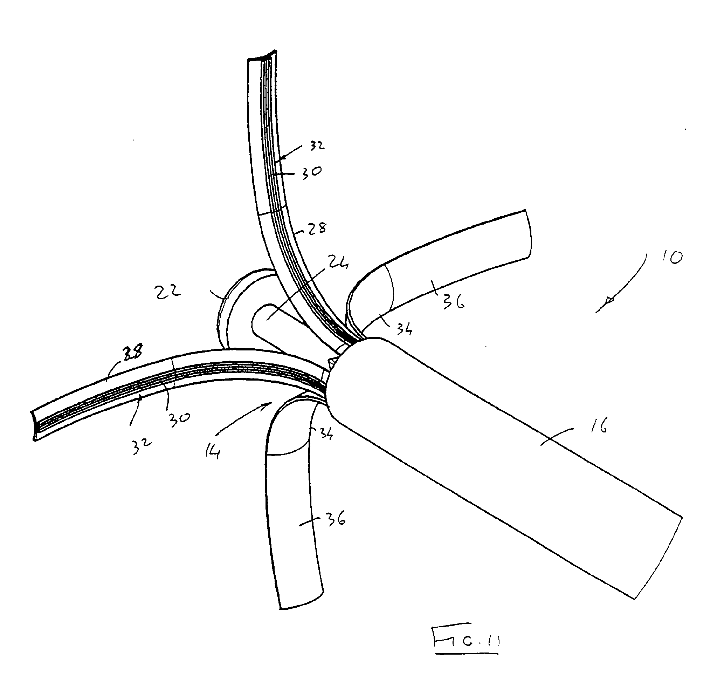

In Fig. 11 of the drawings, another embodiment of a catheter assembly 10 is illustrated. With reference to the previous drawings, like reference numerals refer to like parts, unless otherwise specified.

In this embodiment, the electrodes 36 are carried on the outer surface of their associated carriers 34. It is to be noted that, in Fig. 11 of the drawings, two of the carriers 34 are omitted to illustrate the groups 32 of conductors 30.

The groups 32 of conductors 30 pass through apertures (not shown) in the distal region of the tubular member 14 and run along an operatively outer side of each strut 28 to make electrical and mechanical contact with their associated electrodes 36.

In use, this embodiment of the catheter assembly 10 is used for ablation therapy on the septum between the left and right atria of the heart. The catheter assembly 10 is inserted through the right atrium and punctures the septum through the fossa ovalis in the septum. The sleeve 16 is withdrawn proximally to expose and release the struts 28 so that the carriers 34 adopt the second, splayed or deployed configuration as shown in Fig. 11 of the drawings. Withdrawing the catheter assembly 10 slightly brings the electrodes 36 into contact with tissue on the septum for treating arrhythmogenic foci in the septum.

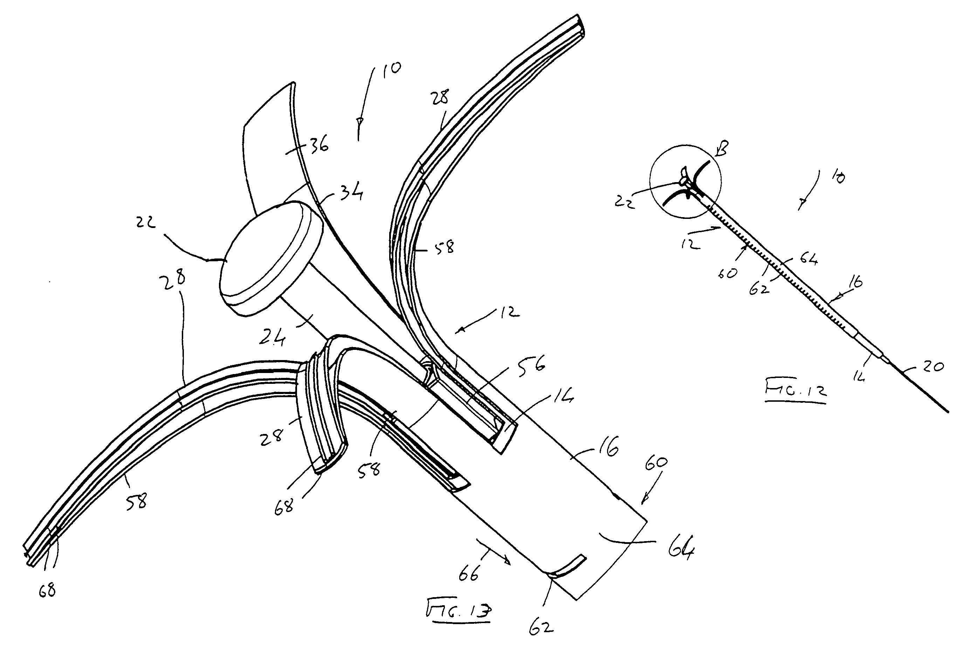

In Figs. 12 and 13 of the drawings, yet a further embodiment of a catheter assembly 10 is illustrated. Once again, with reference to previous drawings, like reference numerals refer to like parts, unless otherwise specified.

In this embodiment, a distal end of the sleeve 16 is crenelated in a similar manner to that of the tubular member 14. Thus, the distal end of the sleeve 16 defines a plurality of discrete components 58. It will be appreciated that there are the same number of components 58 as there are struts 28 of the tubular member 14. Each discrete component 58 lies in register with its associated strut 28. A distal end of each strut 28 is fast with a distal end of its associated component 58. In Fig. 13 of the drawings, three of the carriers 34 are omitted to show the structure of the catheter sheath 12.

Further, in this embodiment, the sleeve 16 defines a deflection zone 60 of the catheter sheath 12, the deflection zone 60 comprising a plurality of longitudinally spaced transverse slots 62. The slots 62 extend approximately two thirds to three quarters of the way about the periphery of the sleeve 16 to leave a longitudinally extending spine region 64 (Figs. 12 and 13) about which the sleeve 16 can deflect. The deflection zone 60 of the sleeve 16 is provided instead of, or in addition to, the deflection zone 40 of the tubular member 14.

In this embodiment, the rest condition of the struts 28, with their associated discrete components 58, is in the first position, i.e. the position in which the struts 28 extend parallel to the longitudinal axis of the tubular member 14. In this configuration, the catheter assembly 10 is inserted through the vasculature of the patient's body and steered to the desired site. At the desired site, the sleeve 16 is urged proximally relative to the tubular member 14 in the direction of arrow 66 (Fig. 13). When this occurs, the struts 28 are withdrawn to their second, splayed configuration as shown in Figs. 12 and 13 of the drawings. Shape memory wires 68 attached to the inner surface of each strut 28 assist in causing the struts 28 to splay outwardly when the sleeve 16 is withdrawn proximally relative to the tubular member 14. It will be appreciated that similar wires could be used in the other embodiments described above either to assist in moving the struts 28 to their operative positions or to withdraw the struts 28 to their inoperative position, as the case may be.

In all the embodiments described above, once the struts 28 are in their splayed or deployed configuration or while they are in the process of being so deployed, the support member 20 is withdrawn proximally relative to the tubular member 14 to bring the end electrode 22 into a position in which it lies substantially in the same plane as the electrodes 36 carried by the struts 28. Selected electrodes 36 and/or 22 can then be used to achieve the desired pattern of ablation at the site in the patient's heart.

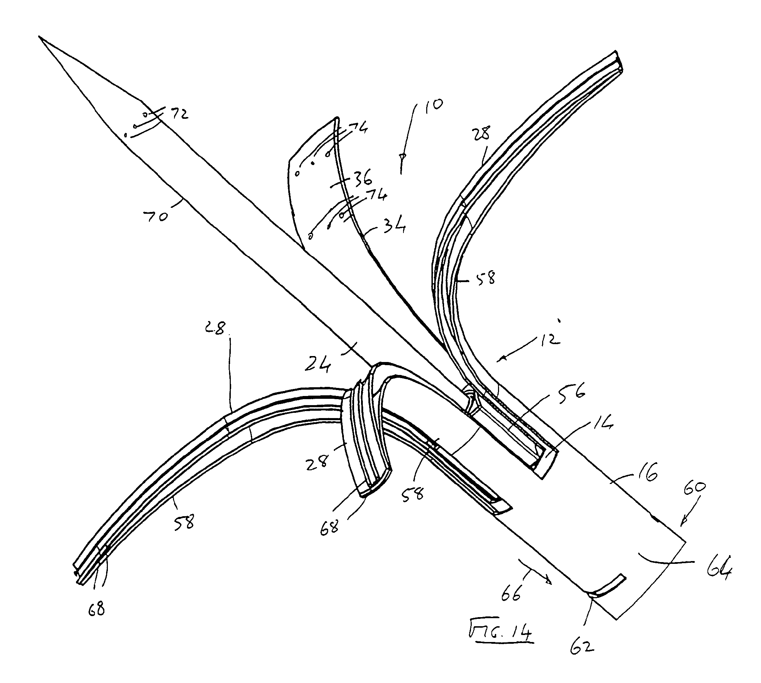

Yet a further embodiment of the catheter assembly 10 is shown in Fig. 14 of the drawings and, once again, with reference to the previous drawings, like reference numerals refer to like parts unless otherwise specified.

In this embodiment, the end electrode is a needle electrode 70 instead of the button electrode 22 of the previous embodiments. The needle electrode 70 is used to achieve ablation within tissue at the site either on its own or in combination with one or more of the electrodes 36. It will be appreciated that, in this embodiment, the support member need not be retractable relative to the catheter sheath 12. When the struts 28 are in their closed position, the carriers 34 envelop the needle electrode 70 and, conversely, when the struts 28 are in their operative position, the needle electrode 70 is exposed and extends distally of the plane in which the electrodes 36 lie.

Further, in this embodiment, the catheter assembly 10 is an irrigation catheter. Hence, ports 72 are provided in the needle electrode 70 through which irrigation fluid is discharged. In addition, ports 74 are defined in the electrodes 36 through which irrigation fluid is discharged. The irrigation ports 72 and 74 are in communication with irrigation conduits (not shown) extending through the lumen of tubular member 14. If desired, the irrigation conduits can be formed as part of the ribbon cable forming the groups 32 of conductors 30. It will also be appreciated that, in the embodiments described above, irrigation can be provided to the electrodes 36 as well as to the end electrode 22, the electrode 22 having appropriate irrigation ports.

It is a particular advantage of the invention that a compact catheter assembly 10 is provided which facilitates being steered through a patient's vasculature. In addition, once the distal region of the catheter assembly 10 is at the desired site in a patient's body, the electrodes 36 can be deployed to define a wide area over which ablation therapy can be effected. Due to the resiliently flexible nature of the struts 28, good tissue/electrode contact results.

In addition, the shape of each carrier 34 lends itself to the application of a relatively long electrode 36 for enabling long, shallow lesions to be formed at the site. Long, shallow lesions result in less trauma but more effective treatment of heart arrhythmias. The use of longer electrodes also means that fewer ablating procedures need to be carried out in the treatment of arrhythmogenic foci. The ability to select electrodes 36 and the electrode 22, 74 also enables different patterns of lesions to be formed with fewer movements of the catheter assembly 10.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A catheter sheath which includes
a tubular member defining a lumen, a distal region of the tubular member defining a plurality of discrete elements, the discrete elements being displaceable between a first position in which the discrete elements extend parallel to a longitudinal axis of the tubular member and a second position in which the discrete elements project outwardly transverse to the longitudinal axis of the tubular member;
a plurality of electrical conductors associated with the tubular member, the conductors projecting into the distal region of the tubular member to be made fast with at least one of the discrete elements;
a sleeve mounted about the tubular member, the sleeve and the tubular member being displaceable axially with respect to each other for effecting displacement of the discrete elements between their first and second positions; and
at least one electrode carried by the at least one of the discrete elements, the at least one electrode being in an operative position when the discrete elements are in their second position.

2. The catheter sheath of claim 1 in which the tubular member is of a settable material so that the discrete elements can be set in one of their first position and their second position as a rest position.

3. The catheter sheath of claim 2 in which the tubular member is mounted on a tube of a flexible material.

4. The catheter sheath of claim 2 or claim 3 in which the rest position of the discrete elements is the second position and the sleeve is axially displaceable relative to the tubular member, displacement of the sleeve towards a distal end of the tubular member displacing the discrete elements to their first position and withdrawal of the sleeve in a proximal direction exposing the discrete elements and allowing the discrete elements to adopt their second position.

5. The catheter sheath of claim 2 or claim 3 in which the rest position of the discrete elements is the first position, the sleeve having complementary discrete components which lie in register with the discrete elements of the tubular member, each discrete element being secured to its associated distal component so that proximal displacement of the sleeve relative to the tubular member causes the discrete elements to be displaced to their second position.

6. The catheter sheath of any one of the preceding claims in which each discrete element is in the form of a strut, each strut supporting at least one electrode, the at least one electrode of each strut being carried on a carrier which is mounted on the strut.

7. The catheter sheath of claim 6 in which the at least one electrode is arranged on an operatively inner surface of its associated carrier so that, when the discrete elements are in their first position, the electrodes are contained within the interior of the tubular member.

8. The catheter sheath of claim 6 in which the at least one electrode is arranged on an operatively outer surface of is associated carrier so that, when the discrete elements are in their first position, the electrodes are contained on an outer surface of the tubular member.

9. The catheter sheath of any one of claims 6 to 8 in which the electrical conductors are arranged in groups of conductors, a group of conductors being associated with each discrete element.

10. The catheter sheath of any one of the preceding claims in which at least one of the tubular member and the sleeve defines a deflection zone arranged proximally of the discrete elements of the tubular member.

11. The catheter sheath of claim 10 which includes an actuator which acts on the deflection zone for controlling deflection of the distal region of the tubular member.

12. A catheter assembly which includes
a catheter sheath as claimed in any one of the preceding claims; and
a support member arranged within the lumen of the tubular member.

13. The catheter assembly of claim 12 which includes an end electrode carried on a distal end of the support member and arranged in alignment with the distal end of the tubular member, the support member being displaceably arranged in the lumen of the tubular member, the arrangement being such that, when the discrete elements of the tubular member are in their first position, the end electrode is arranged distally of the discrete elements and, when the discrete elements are in their second position, the support member is displaced proximally so that the end electrode lies substantially in a plane in which the discrete elements lie.

14. The catheter assembly of claim 13 in which a flexible cylindrical member is interposed between the end electrode and the distal end of the support member to create increased flexibility at the distal region of the catheter sheath.

15. The catheter assembly of any one of claims 12 to 14 in which a seal is arranged about the support member in the region of the discrete elements of the tubular member so that, when the discrete elements of the tubular member are in their first position, they seat about the seal to inhibit the ingress of foreign matter into the lumen of the tubular member.
